Europäisches Patentamt

European Patent Office

Office européen des brevets

(11) Veröffentlichungsnummer: **0 097 846**
**B1**

(12) # EUROPÄISCHE PATENTSCHRIFT

(45) Veröffentlichungstag der Patentschrift:
26.10.88

(51) Int. Cl.⁴: **A 61 L 15/01**

(21) Anmeldenummer: **83105563.7**

(22) Anmeldetag: **07.06.83**

---

(54) **Verbandmaterial auf Hydrogelbasis und Verfahren zu dessen Herstellung.**

---

(30) Priorität: **30.06.82 DE 3224382**

(43) Veröffentlichungstag der Anmeldung:
**11.01.84 Patentblatt 84/2**

(45) Bekanntmachung des Hinweises auf die Patenterteilung:
**26.10.88 Patentblatt 88/43**

(84) Benannte Vertragsstaaten:
**AT CH DE FR GB IT LI NL SE**

(56) Entgegenhaltungen:
**EP - A - 0 022 064
EP - A - 0 040 862
EP - A - 0 095 892
EP - A - 2 693 438**

(73) Patentinhaber: **Beiersdorf Aktiengesellschaft,
Unnastrasse 48, D-2000 Hamburg 20 (DE)**

(72) Erfinder: **Hofeditz, Wolfgang, Dr., Vogt-Wells-Kamp 14,
D-2000 Hamburg 54 (DE)**
Erfinder: **Hornig, Joachim, Friedrich Ebert Strasse 87,
D-2000 Hamburg 61 (DE)**
Erfinder: **Karmann, Werner, Dr., Stolpmünder Strasse 11,
D-2000 Hamburg 73 (DE)**

---

## Beschreibung

Die Erfindung betrifft ein neues Verbandmaterial auf Basis eines transparenten, wasserhaltigen, flexiblen Polyvinylalkohol-Gels, welches vorzugsweise in Folienform vorliegt und gegebenenfalls mit einer oder mehreren Schichten eines anderen Trägermaterials als Zwischen- und/oder Deckschicht kombiniert sein kann sowie das Verfahren zur Herstellung des Verbandmaterials.

Es sind aus der Patentliteratur bereits Hydrogele und hydrogelhaltige Verbandstoffe aus den unterschiedlichsten Gelbildnern bekannt, wie beispielsweise aus vinylgruppenvernetzten Polyäthylenoxiden, Polyurethanharnstoffen, Polysiloxanen, Mischungen aus gelierfähigen Polysacchariden und/oder Proteinen mit hydrophilen Polymerisaten oder Pfropfpolymeren aus hydrolysierter Stärke und Polyacrylnitril. Der Nachteil dieser Gele ist zumeist ihre geringe mechanische Festigkeit in gequollenem Zustand, so dass sie bei mechanischer Beanspruchung zerkrümeln und zerfallen. Obwohl insbesondere zur Behandlung von Brandwunden und ähnlichen grossflächigen Wunden ein grosses Bedürfnis nach derartigen Verbandmitteln besteht, haben sie deshalb bisher gar nicht oder nur sehr beschränkt Eingang in die Praxis gefunden.

Es ist weiterhin bekannt, dass wasserlösliche Polyvinylalkoholfolien durch Behandeln mit Aldehyden wie Formaldehyd, Glyoxal, Glutardialdehyd gehärtet und dadurch wasserunlöslich gemacht werden können. Ferner sind Schaumstoffe auf Basis von formaldehydvernetztem Polyvinylalkohol bekannt. Sie werden wegen ihrer guten physiologischen Verträglichkeit in der Wundbehandlung eingesetzt. Diese Folien und Schäume sind in trockenem Zustand jedoch hart und nicht flexibel.

Gele, die aus Polyvinylalkohol, einem Polyol und Wasser bestehen und eventuell auch ein wasserlösliches Polymer enthalten, sind z.B. aus US-A-2 693 438 und EP-A-95892 bekannt.

Aufgabe der Erfindung war daher die Entwicklung eines Verbandmaterials bestehend aus mindestens einer Schicht einer elastischen, transparenten, in Wasser quellbaren aber unlöslichen, physiologisch sehr gut verträglichen Gelfolie, die sowohl in trockenem als auch in gequollenem Zustand eine hohe mechanische Stabilität und hohe Flexibilität besitzt, mit gegebenenfalls zusätzlich anderen stützenden oder saugenden Materialien als Zwischen- oder Deckschicht.

Gelöst wird diese Aufgabe durch ein Verbandmaterial, das aus mindestens einer Schicht eines polymeren, hydrophilen Gels und gegebenenfalls einer oder mehrerer Schichten eines Trägermaterials als Zwischen- und/oder Deckschicht besteht und dadurch gekennzeichnet ist, dass das Gel aus

    40–50 Gew.-% eines mit Formaldehyd vernetzten und acetalisierten, in Wasser zumindest 90% unlöslichen Polyvinylalkohols

15–30 Gew.% Wasser

15–45 Gew.% eines mehrwertigen Alkohols mit 2–6 C-Atomen sowie gegebenenfalls geringen Mengen an Zusatz- und/oder Hilfsstoffen besteht.

Als Ausgangsmaterial für die Herstellung des in kaltem und heissem Wasser praktisch nicht mehr löslichen, d.h. höchstens noch zu 5–10% löslichen, mit Formaldehyd weitgehend vernetzten bzw. acetalisierten Polyvinylalkohols, wird ein Polyvinylalkohol eingesetzt mit einem Verseifungsgrad von 85–90%, vorzugsweise 88%, der als 4%ige wässrige Lösung bei 20 °C eine Viskosität von 3–40 mPa.s (3–40 cP), vorzugsweise etwa 18 mPa.s (18 cP), aufweist.

Als mehrwertige Alkohole, die als Weichmacher für die Folie wirken, eignen sich besonders solche mit 2–6 C-Atomen und davon insbesondere Äthylenglycol, Di-Glycol, Tri-Glycol, Glyzerin, Propandiol, Butandiol, Hexandiol oder Hexantriol. Die Alkohole können einzeln oder im Gemisch in Mengen von 15–45 Gew.%, vorzugsweise 20–30 Gew.%, in dem Gel enthalten sein je nach der gewünschten Flexibilität der Gelfolie. Als besonders vorteilhaft hat sich dabei 1.2-Propandiol erwiesen.

Unter den Zusatz- und/oder Hilfsstoffen, welche in geringen Mengen in dem Gel enthalten sein können, sind in erster Linie medizinische Wirkstoffe wie Antibiotika, beispielsweise Gentamycin oder PVP-Jod, physiologisch unbedenkliche Farbstoffe, Pigmente, Duftstoffe, Füllstoffe in Form von Fasern oder Pulvern, oder Konservierungsmitteln zu verstehen.

Das erfindungsgemässe weiche und flexible Gel wird in der Weise hergestellt, dass man den noch unvernetzten Polyvinylalkohol (PVAL) in Wasser löst, die Lösung mit einer Säure, vorzugsweise Salzsäure, ansäuert, eine wässrige Formaldehyd-Lösung in der Menge zugibt, dass nach Reaktionsende kein freier Aldehyd mehr nachweisbar ist und die Lösung mehrere Stunden bei 50–80 °C, vorzugsweise 60–70 °C, reagieren d.h. vorvernetzen lässt, wobei das Reaktionsprodukt (Präpolymer) als gelartige Masse anfällt. Nach Entfernen der Salzsäure, vorteilhafter Weise durch Neutralisation mit Natronlauge und anschliessendem mehrfachem Auswaschen mit Wasser erhält man eine plastisch weiche, wasserhaltige Masse, in die sich die genannten Zusatzstoffe sowie die Weichmacher durch Untermischen leicht einarbeiten lassen.

Das vorvernetzte Gel wird in der gewünschten Schichtstärke, in der Regel etwa 0,5 bis wenige mm dick, auf einem Hilfsträger, beispielsweise einem silikonisierten Kraftpapier, ausgestrichen oder ausgegossen und anschliessend durch eine weitere thermische Behandlung (etwa eine Stunde lang bei 80–100 °C) getrocknet bis auf einen Restwasser-Gehalt von 15–30 Gew.%, vorzugsweise ca. 25 Gew.-%, und gleichzeitig dabei nachvernetzt.

Die auf diese Weise erhaltenen Gelfolien sind schmiegsam und gummi-elastisch durch den Weichmacher- und Wassergehalt; sie sind reissfest und praktisch wasserunlöslich durch die starke Vernetzung und besitzen dabei noch ein Wasseraufnahmevermögen von etwa 600–800%, was für ihre Verwendbarkeit als Verbandmittel besonders wichtig ist. Ausserdem sind die Folien transparent, so dass die Wunde durch sie hindurch beobachtet werden kann, jedoch undurchlässig für Bakterien. Feuchtigkeit kann durch sie in Dampfform hindurchdiffundieren.

Die erfindungsgemässen Gelfolien können als solche auf die Wunde aufgelegt werden, da sie eine ausreichende Sekretaufnahmefähigkeit und Reissfestigkeit besitzen, vorzugsweise werden sie jedoch mit einem oder mehreren weiteren Trägermaterialien zu einem Verbundprodukt verarbeitet und in dieser Form verwendet. Besonders geeignet hierfür sind feine Gewebe oder Vliese, auf beispielsweise Polyamid-, Polyurethan- oder Polyester-Basis, oder offenporige Schaumstoffe, die in das Gel bei der Herstellung eingebettet werden, oder stärker saugende Materialien in Form von Schaumstoffen, Geweben, Gewirken oder Vliesen auf Kunststoff- oder Naturstoff-Basis, die auf die Gelfolie aufkaschiert werden. Auch schützende, gegebenenfalls wasserdampfundurchlässige Folien können als Deckschicht auf dem Gel verankert sein.

Diese weiteren Trägerschichten werden vorteilhafterweise in oder auf die vorvernetzte Gelschicht, auf welcher sie gut haften, kaschiert und bei der folgenden Nachvernetzung noch fester mit dieser verbunden.

Die fertigen Verbandmaterialien können anschliessend in gewünschter Weise konfektioniert und vorzugsweise in wasserdampfundurchlässige Beutel eingesiegelt sowie beispielsweise durch γ-Strahlung sterilisiert werden.

Die erfindungsgemässen elastischen und wasserhaltigen Polyvinylalkoholfolien eignen sich in besonderer Weise für bisher immer noch schwierig zu versorgende Wunden, wie grossflächige Brand- und Schürfwunden, oder auch zur Abdeckung von Hautentnahmestellen. Sie wirken dabei wie eine synthetische Haut oder Membran, die ein optimales Milieu schafft, indem sie den Wundgrund feucht aber nicht nass hält und durch langsames Abdiffundieren von überschüssiger Feuchtigkeit eine für die Heilung günstige Temperatur herstellt. Gleichzeitig schützt sie die Wunde vor äusserer, auch bakterieller Kontamination. Wie Versuche gezeigt haben, bildet sich unter der Abdeckung normales Granulationsgewebe mit Kapillareinsprossung und gerichteten Kollagenfasern. Ist das Gel noch mit Wirkstoffen dotiert, beispielsweise 0,25–0,5 Gew.-% Gentamycin oder 0,1–0,15 Gew.-% PVP-Jod, so wirkt sie dadurch zusätzlich bakterizid und damit heilungsfördernd.

Die folgenden Beispiele sollen die Herstellung der erfindungsgemässen Verbandmaterialien näher erläutern.

Beispiel 1

416,5 g einer wässrigen Polyvinylalkohol-Lösung (12%ig, Verseifungsgrad ca. 88%, Viskosität einer 4%igen wässrigen Lösung bei 20°C: 18 mPa.s (18 cP)) und 96,5 g Salzsäure (32%ig) werden in einem Planschliffkolben mit Rührer vorgelegt, auf 70°C aufgeheizt und 14 g wässrige Formaldehydlösung (35%ig) zugetropft.

Man lässt drei Stunden bei dieser Temperatur reagieren, neutralisiert mit Natronlauge und wäscht danach die gelartig ausgefallene Masse mit heissem Wasser gründlich aus. Der vorvernetzte Polyvinylalkohol wird vom überschüssigen Waschwasser abgetrennt und 20 Gew.-% Glyzerin eingearbeitet. In dieser Stufe lassen sich auch zusätzliche Farb-, Wirk- und andere Zusatzstoffe untermischen, wenn diese gewünscht sind.

Die homogene weiche Masse wird anschliessend auf Trennpapier (silikonisiertem Kraftpapier) in der gewünschten Strichstärke (etwa 0,5–2 mm) ausgestrichen und im Heissluftschrank bei 80–90°C 1 Stunde getrocknet und nachvernetzt.

Die resultierende Folie hat eine Restfeuchte von 10–15%, ein Wasseraufnahmevermögen von etwa 600–800% und eine Wasserabdampfrate von 35–40 mg/cm² 24 h im gequollenen Zustand.

Sie ist sowohl trocken als auch gequollen transparent, zähelastisch und mechanisch sehr stabil.

Beispiele 2–4

Man arbeitet wie in Beispiel 1 beschrieben, es werden jedoch anstatt Glyzerin als Weichmacher 25% Propan-1.2-diol, oder zusätzlich zum Propandiol 6% Aluminium-Pulver oder 2% PVP-Jod in das ausgefällte Präpolymere eingearbeitet.

Auch diese Gelfolien sind geschmeidig, zähelastisch und je nach Weichmachergehalt mehr oder weniger weich.

Beispiele 5–6

Auf die ausgestrichene Schicht einer vorvernetzten, noch feuchten Masse gemäss Beispiel 2, d.h. mit einem Weichmacher-Gehalt von 25% Propan-1.2-diol, wird ein Vlies oder ein offenporiger Schaumstoff aus Polyäthylen, Polypropylen, Polyurethan oder Polyester aufkaschiert und anschliessend dem Trocknungs- bzw. Nachvernetzungsprozess unterworfen.

Es entsteht jeweils ein stark feuchtigkeitsaufnahmefähiges Verbundprodukt mit den Eigenschaften der kombinierten Materialien.

Beispiel 7

Ein dünnes Polyamidgewebe wird mit der vorvernetzten Masse gemäss Beispiel 2 beschichtet und durchtränkt, so dass dieses nach dem Trocknen und Nachvernetzen in der Gelfolie eingeschlossen ist und zur inneren Verfestigung der Folie dient.

**Patentansprüche**

1. Verbandmaterial aus mindestens einer Schicht eines polymeren, hydrophilen Gels und gegebenenfalls einer oder mehrerer Schichten eines Trägermaterials als Zwischen- und/oder Deckschicht, dadurch gekennzeichnet, dass das Gel aus
40–50 Gew.-% eines mit Formaldehyd vernetzten und acetalisierten, in Wasser zu mindestens 90% unlöslichen Polyvinylalkohols,

15–30 Gew.-% Wasser

15–45 Gew.-% eines oder mehrerer mehrwertigen Alkohole mit 2–6 C-Atomen

sowie gegebenenfalls geringen Mengen an Zusatz- und/oder Hilfsstoffen besteht.

2. Verbandmaterial gemäss Anspruch 1, dadurch gekennzeichnet, dass der Polyvinylalkohol vor seiner Umsetzung mit Formaldehyd einen Verseifungsgrad von 85–90%, vorzugsweise 88%, und eine Viskosität einer 4%igen wässrigen Lösung bei 20 °C von 3–40 mPa.s (3–40 cP), vorzugsweise (18 mPa.s) (18 cP), aufweist.

3. Verbandmaterial gemäss Anspruch 1 oder 2, dadurch gekennzeichnet, dass das Gel die mehrwertigen Alkohole Äthylenglycol, Di-Glycol, Tri-Glycol, Glyzerin, Propandiol, Butandiol, Hexandiol oder Hexantriol einzeln oder im Gemisch enthält.

4. Verbandmaterial gemäss Anspruch 3, dadurch gekennzeichnet, dass das Gel 20–30 Gew.-% Propan-1,2-diol enthält.

5. Verbandmaterial gemäss einem oder mehreren der Ansprüche 1–4, dadurch gekennzeichnet, dass die Gelschicht mit Zwischen- und/oder Deckschichten aus Geweben, Gewirken, Vliesen oder Schaumstoffen auf Synthese- oder Naturstoff-Basis zu einem Verbundprodukt kombiniert ist.

6. Verfahren zur Herstellung von flexiblen Gelschichten gemäss einem oder mehreren der Ansprüche 1–5, dadurch gekennzeichnet, dass 40–50 Gew.-% (jeweils bezogen auf das Endprodukt) Polyvinylalkohol in Wasser gelöst, mit Formaldehyd bei erhöhter Temperatur umgesetzt, in das ausgefallene und ausgewaschene Reaktionsprodukt (Präpolymer) 15–45 Gew.-% eines oder mehrerer mehrwertigen Alkohole mit 2–6 C-Atomen sowie gegebenenfalls geringe Mengen an weiteren Zusatz- oder Hilfsstoffen eingearbeitet, die weiche Masse blatt- oder bahnförmig in der gewünschten Dicke ausgestrichen und anschliessend einer weiteren etwa einstündigen Wärmebehandlung bei ca. 80–100 °C zur Trocknung auf einen Rest-Wassergehalt von 15–30 Gew.-% und gleichzeitigen Nachvernetzung unterworfen wird.

7. Verfahren gemäss Anspruch 7, dadurch gekennzeichnet, dass die Gelschicht mit Zwischen- und/oder Deckschichten versehen wird, indem in die ausgestrichene Schicht des vorvernetzten Gels eine Bahn aus Gewebe, Vlies, Folie oder offenporigem Schaumstoff auf Natur- oder Kunststoff-Basis eingebettet oder auf diese Schicht aufkaschiert wird und das Verbundprodukt anschliessend der Wärmebehandlung zur Trocknung und Nachvernetzung unterworfen wird.

## Claims

1. Dressing material consisting of at least one layer of a polymeric, hydrophilic gel and, if desired, of one or more layers of a carrier material as intermediate and/or top layer, characterized in that the gel consists of

40–50% by weight of a polyvinyl alcohol which is crosslinked and acetalized with formaldehyde and is insoluble in water to at least 90%,

15–30% by weight of water

15–45% by weight of one or more polyhydric alcohols having 2–6 C atoms

and, if desired, small amounts of additives and/or auxiliaries.

2. Dressing material according to Claim 1, characterized in that the polyvinyl alcohol before its reaction with formaldehyde has a degree of hydrolysis of 85–90%, preferably 88%, and a viscosity of a 4% strength aqueous solution at 20 °C of 3–40 mPa.s (3–40 cP), preferably 18 mPa.s (18 cP).

3. Dressing material according to Claim 1 or 2, characterized in that the gel contains the polyhydric alcohols ethylene glycol, diethylene glycol, triethylene glycol, glycerol, propanediol, butanediol, hexanediol or hexanetriol individually or in a mixture.

4. Dressing material according to Claim 3, characterised in that the gel contains 20–30% by weight of propane-1,2-diol.

5. Dressing material according to one or more of Claims 1–4, characterized in that the gel layer is combined with intermediate and/or top layers made of woven fabrics, knitted fabrics, nonwovens or foam materials based on synthetic or natural materials to give a composite product.

6. Process for the preparation of flexible gel layers according to one or more of Claims 1–5, characterized in that 40–50% by weight (in each case relative to the end product) of polyvinyl alcohol dissolved in water are reacted with formaldehyde at elevated temperature, 15–45% by weight of one or more polyhydric alcohols having 2–6 atoms and, if desired, small amounts of further additives or auxiliaries are incorporated into the precipitated and thoroughly washed reaction product (prepolymer), the soft material is spread out in sheet or web form in the desired thickness and subsequently subjected to a further heat treatment for approximately one hour at about 80–100 °C for the purpose of drying down to a residual water content of 15–30% by weight and of simultaneous additional crosslinking.

7. Process according to Claim 7, characterized in that the gel layer is provided with intermediate and/or top layers by embedding a sheet made of woven fabric, nonwoven, film or open-cell foam material based on natural or synthetic materials in the spread-out layer of the previously crosslinked gel or laminating onto this layer and subsequently subjecting the composite product to the heat treatment for the purpose of drying and additional crosslinking.

## Revendications

1. Pansement formé d'au moins une couche d'un gel polymère hydrophile et éventuellement d'une ou plusieurs couches d'une matière de support comme couche intermédiaire et/ou de couverture, caractérisé en ce que le gel consiste en 40–50% en poids d'un poly(alcool vinylique) réticulé et acétalysé par le formaldéhyde et insoluble pour au moins 90% dans l'eau

15–30% en poids d'eau
15–45% en poids d'un ou plusieurs alcools polyvalents de 2 à 6 atomes de carbone et
éventuellement en faibles quantités d'additifs et/ou auxiliaires.

2. Pansement suivant la revendication 1, caractérisé en ce que le poly(alcool vinylique), avant sa réaction avec le formaldéhyde, a un degré de saponification de 85–90% et de préférence de 88% et une viscosité en solution aqueuse à 4% à 20°C de 3–40 mPa.s (3–40 cP) et de préférence de 18 mPa.s (18 cP).

3. Pansement suivant la revendication 1 ou 2, caractérisé en ce que le gel contient isolément ou en mélange les alcools polyvalents ci-après éthylèneglycol, diglycol, triglycol, glycérine, propanediol, butanediol, hexanediol ou hexanetriol.

4. Pansement suivant la revendication 3, caractérisé en ce que le gel contient 20–30% en poids de propane-1,2-diol.

5. Pansement suivant une ou plusieurs des revendications 1–4, caractérisé en ce que la couche de gel est combinée en un produit mixte avec des couches intermédiaires et/ou de couverture formées de tissus, de tricots, de voiles ou de mousses à base de matière synthétique ou naturelle.

6. Procédé de fabrication de couches de gel flexibles suivant une ou plusieurs des revendications 1–5, caractérisé en ce qu'on dissout 40–50% en poids (chacun sur base du produit final) de poly(alcool vinylique) dans de l'eau, on le fait réagir avec du formaldéhyde à température élevée, on incorpore au produit de réaction (prépolymère) précipité et lavé 15–45% en poids d'un ou plusieurs alcools polyvalents de 2 à 6 atomes de carbone ainsi qu'éventuellement de faibles quantités d'autres additifs ou auxiliaires, on étale la masse molle en forme de feuille ou de nappe en l'épaisseur souhaitée et on la soumet ensuite à un traitement thermique supplémentaire d'environ une heure à environ 80–100°C pour le séchage jusqu'à une teneur en eau résiduelle de 15–30% en poids et pour la post-réticulation simultanée.

7. Procédé suivant la revendication 6, caractérisé en ce qu'on munit la couche de gel de couches intermédiaires et/ou de couverture en noyant dans la couche étalée du gel préréticulé une nappe de tissu, de voile, de feuille ou de mousse à pores ouverts à base de matière naturelle ou synthétique ou en l'appliquant en doublure sur cette couche et en soumettant le produit mixte ensuite à un traitement thermique pour le séchage et la post-réticulation.